# EUROPEAN PATENT APPLICATION

(11) **EP 1 900 746 A1**
(43) Date of publication of application: **19.03.2008**
(21) Application number: 06780752.9
(22) Date of filing: 03.07.2006
(51) Int. Cl.: C07H 17/08, A61K 31/7048, A61P 35/00

(54) **VERSIPELOSTATIN DERIVATIVE, ANTI-CANCER AGENT AND PROCESSES FOR PRODUCTION OF THE DERIVATIVE AND AGENT**

(30) Priority: 01.07.2005 JP 2005194412
(71) Applicant: TOUDAI TLO, Ltd., Tokyo 113-0033 (JP)
(72) Inventor: SHIN-YA, Kazuo, 1130022 (JP)
(74) Representative: Smaggasgale, Gillian Helen
(86) International application number: PCT/JP2006/313248
(87) International publication number: WO 2007/004621

(57) **Abstract**

Provided is a new anti-cancer agent lower in cell toxicity.

The present invention provides the compounds represented by the general formula of "Formula 1" or the salt thereof. The compounds are compounds having an organic group introduced on a particular ketone group of VST that functions to inhibit induction of GRP 78 expression, similarly to VST, and is applicable, for example, as an anti-cancer agent. The compounds are also advantageous in that it is less toxic than VST and has lower adverse reaction.

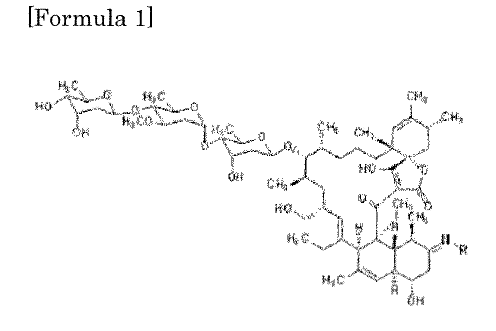

## Description

### TECHNICAL FIELD

The present invention relates to the new compounds, a pharmaceutical composition such as anti-cancer agent and methods of producing the same. More specifically, it relates to a versipelostatin derivative prepared by converting part of the chemical structure of versipelostatin that has an anti-cancer activity consistent as or higher than and a toxicity lower than versipelostatin, a pharmaceutical composition, as anti-cancer agent, containing the same such, and methods of producing the same.

### BACKGROUND ART

GRP 78 has been attracting attention as a target of anti-cancer agent. GRP 78 (Glucose-Regulated Protein: 78 kD) is a protein called HSPA5 (Heat-Shock 70-kD Protein 5) or BIP (immunoglobulin heavy chain-Binding Protein), and it is also a stress-responding molecular chaperone belonging to the HSP 70 family that is present in endoplasmic reticulum. Expression of GRP 78 is known to be induced, for example, under glucose-deficient condition in animal cell (see, for example, Nonpatent Literature 1).

Differently from normal tissues, generally, solid cancers are known to have a region in the glucose-deficient state (see for example Nonpatent Literature 2), and expression of GRP 78 was also reported to be induced (see for example Nonpatent Literature 3). In addition, it was also reported that induction of GRP 78 expression resulted in inhibition of apoptosis induction, for example, under the glucose-deficient condition (see for example Nonpatent Literature 4). These findings suggest that it may be possible to induce apoptosis by inhibition of induction of GRP 78 expression in the glucose-deficient state, i.e., to kill the cell (cancer cell) in the deficient state by inhibition of induction of GRP 78 expression. For these reasons, intensive studies for the substance inhibiting induction of GRP 78 expression as a new anti-cancer agent is in progress (see for example Nonpatent Literatures 2 to 7).

The inventors had earlier found versipelostatin (hereinafter, referred to as "VST") as a substance inhibiting induction of GRP 78 expression (see Patent Document 1). The substance is a tetronic acid derivative having the Chemical Formula represented by the following "Formula 8", and the inventors isolated and purified it from the supernatant of a culture of a fungus, Streptomyces versipellis 4083-SVS6, which was isolated from a soil collected in Miyoshi City, Hiroshima, Japan. As described above, VST has an action to inhibit induction of GRP 78 expression and may be applicable as an anti-cancer agent.

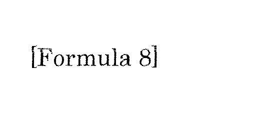

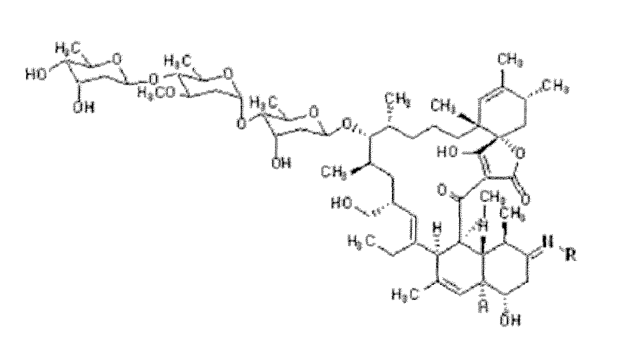

Patent Document 1: WO 2003/044209
Nonpatent Literature 1: Biochemistry Dictionary, 3rd Ed. (Tokyo Kagaku Dozin): p.422 (glucose-regulating protein), p.607 (GRP94)
Nonpatent Literature 2: Tomida A. et al., "Drug resistance mediated by cellular stress response to the microenvironment of solid tumors ", Anticancer Drug Des. (2): 169-77 (1999)
Nonpatent Literature 3: Jamora C. et al., "Inhibition of tumor progression by suppression of stress protein GRP 78/Bip induction in fibrosarcoma B/C10 ME", Proc. Natl. Acad. Sci. USA, (15): 7690-4 (1996)
Nonpatent Literature 4: Miyake H., et al., "Stress protein GRP 78 prevents apoptosis induced by calcium ionophore, ionomycin, in human prostate cancer cells", J. Cell Biochem. (3): 396-408 (2000)
Nonpatent Literature 5: Hae-Ryong Park, et al., "Versipelostatin, a novel GRP 78/Bip molecular chaperone down-regulator of microbial origin", Tetrahedron Letters (43): 6941-6945 (2002)
Nonpatent Literature 6: Park H. R., et al., "Effect on tumor cells of blocking survival response to glucose deprivation", J. Natl. Cancer Inst. (17): 1300-10 (2004)
Nonpatent Literature 7: Lee A. S., et al., "The glucose-regulated protein: stress induction and clinical application", Trends Biochem. Sci., (8): 504-10 (2001)

### Disclosure of the Invention

### Problems to be Solved by the Invention

A main object of the present invention is to provide a new anti-cancer agent having anti-cancer activity that is lower in toxicity.

### Means to Solve the Problems

The inventors have found that it was possible to retain or enhance the anti-cancer activity of VST and yet reduce the toxicity of VST by introducing an organic group on a particular ketone group in the VST chemical structure. Thus, the present invention provides the compounds represented by the general formula of "Formula 9" or the salt thereof, wherein R represents an organic group.

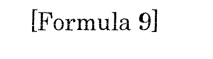

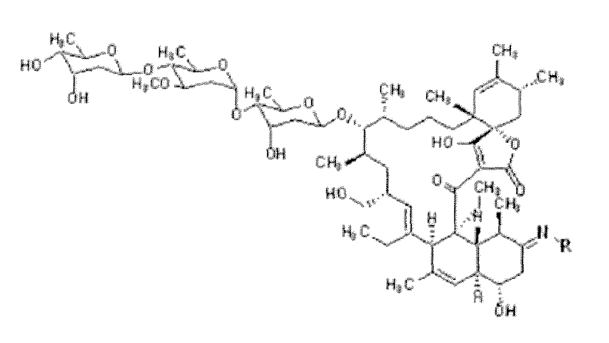

The compounds have an action to inhibit induction of GRP 78 expression by microsomal stress, and are less toxic than VST. In addition, the compounds inhibit GRP 78 expression in cell in the glucose-deficient state, similarly to VST, but are highly probably inactive to cells wherein the glucose metabolism is in the normal state. Accordingly, a pharmaceutical composition containing the compounds according to the present invention may possibly be used, for example, as an anti-cancer agent that has smaller adverse reaction and is higher in stability

### Effects of the Invention

It is possible to inhibit induction of GRP 78 expression by using the compounds according to the present invention and also to reduce the toxicity of VST.

### BEST MODE OF CARRYING OUT THE INVENTION

### <Compounds according to the present invention>

The compounds according to the present invention include widely the compounds represented by the general formula of "Formula 9" above and the pharmaceutically allowable salts thereof.

In the general formula of "Formula 9", R represents an organic group. The organic groups R include widely carbon skeleton-containing groups (including groups having one or more of oxygen, nitrogen and sulfur atoms in the skeleton), the derivatives thereof having the chemical structures other than the skeleton at one or more sites additionally modified, and the like.

The organic group R is preferably a group represented by -O-R¹ (wherein, R¹ represents an organic group), (general formula of "Formula 10").

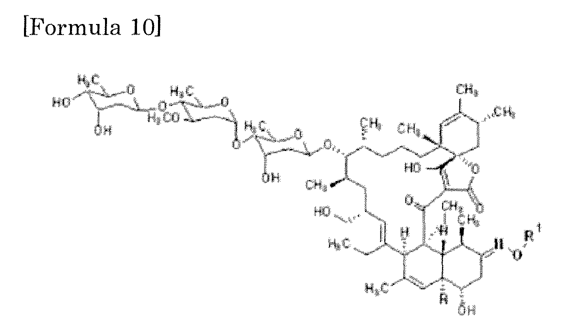

Similarly to the group above, the organic groups R¹ include widely carbon skeleton-containing groups (including groups having one or more of oxygen, nitrogen and sulfur atoms in the skeleton), the derivatives thereof having the chemical structures other than the skeleton at one or more sites additionally modified, and the like.

R¹ is preferably a hydrocarbon group (that may be substituted). Examples of the substituent groups of the hydrocarbon group include amino groups (that may be substituted), heterocyclic groups (that may be substituted), alicyclic groups (that may be substituted), and the like. The compounds according to the present invention include those substituted with one or more substituents.

In particular, the compounds having a structure represented by -(CH₂)ₙ-R² is more preferably as R¹. In the Formula, n is an integer of 1 or more, and R² represents a hydrogen atom, an amino group (that may be substituted), a heterocyclic group (that may be substituted), or an alicyclic hydrocarbon group (that may be substituted).

R² is preferably an acylamino group (that may be substituted), a phenyl group (that may be substituted), a morpholinyl group (that may be substituted), or a triazinyl group (that may be substituted).

The hydrocarbon group according to the present invention will be described below.

The hydrocarbon group according to the present invention is, for example, (1) a linear aliphatic hydrocarbon group, (2) an alicyclic hydrocarbon group, or (3) an aryl group.

(1) The linear aliphatic hydrocarbon group is, for example, a straight-chain or branched aliphatic hydrocarbon group such as alkyl, alkenyl, or alkynyl.

Examples of the alkyl groups include methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, 1-methylheptyl, 1-ethylhexyl, n-octyl, 1-methylheptyl, nonyl and the like.

Examples of the alkenyl groups include vinyl, allyl, isopropenyl, 2-methylallyl, 1-propenvi, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like.

Examples of the alkynyl groups include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like.

(2) The alicyclic hydrocarbon group is, for example, a saturated or unsaturated alicyclic hydrocarbon group such as cycloalkyl, cycloalkenyl, or cycloalkadienyl group.

Examples of the cycloalkyl groups include cyclopropyl, cyclobutyl, cyclopentyl, cyclohexyl, cycloheptyl, cyclooctyl, cyclononyl and the like.

Examples of the cycloalkenyl groups include 2-cyclopenten-1-yl, 3-cyclopenten-1-yl, 2-cylcohexen-1-yl, 3-cylcohexen-1-yl, 1-cyclobuten-1-yl, 1-cyclopenten-1-yl and the like.

Examples of the cycloalkadienyl groups include 2,4-cyclopentanedien-1-yl, 2,4-cyclohexanedien-1-yl, 2,5-cyclohexanedien-1-yl and the like.

(3) The aryl group is, for example, a monocyclic or fused polycyclic aromatic hydrocarbon group.

Examples of the aromatic hydrocarbon groups include phenyl, naphthyl, anthryl, azulenyl, phenanthryl, phenalenyl, fluorenyl, indacenyl, biphenylenyl, heptalenyl, acenaphthylenyl and the like.

Hereinafter, the substituent groups on the hydrocarbon groups will be described in detail.

The substituent groups on the hydrocarbon group according to the present invention include, for example, (1) amino groups (that may be substituted), (2) heterocyclic groups (that may be substituted), (3) alicyclic groups (that may be substituted) and the like.

(1) The amino groups include an amino group as well as substituted amino groups. The amino group is not limited to be a group substituted only at one substituent and thus, may be substituted with one to three substituents groups.

Examples of substituent groups on the amino group include lower alkyl groups (e.g., C1-6 alkyl groups such as methyl, ethyl, propyl, isopropyl, butyl, isobutyl, tert-butyl, pentyl, and hexyl), and acyl groups (e.g., C1-6 alkanoyl groups such as formyl, acetyl, propionyl and pivaloyl, benzoyl, C1-6 alkylsulfonyl groups such as methanesulfonyl, and benzenesulfonyl). These lower alkyl groups also include those substituted with one or more of halogen atoms (such as fluorine, chlorine, bromine, and iodine), C1-6 alkoxy groups (such as methoxy, ethoxy, trifluoromethoxy, 2,2,2-trifluoroethoxy, trichloromethoxy, and 2,2,2-trichloroethoxy that may be halogenated), and C7-11 alkylaryl groups (such as o-toluyl, m-toluyl, p-toluyl, xylyl, mesityl, and C1-5 alkyl-phenyl).

Examples of the other substituent groups on the amino group include C1-6 alkoxycarbonyl groups (such as methoxycarbonyl, ethoxycarbonyl, trifluoromethoxycarbonyl, 2,2,2-trifluoroethoxycarbonyl, trichloromethoxycarbonyl, and 2,2,2-trichloroethoxycarbonyl that may be halogenated), C1-6 alkoxycarbonyl groups (such as benzyloxycarbonyl that may be substituted with phenyl groups), aryl groups (such as phenyl, 1-naphthyl, and C6-10 aryls (such as 2-naphthyl)), aralkyl groups (such as benzyl, C7-10 aralkyls (such as phenethyl), and phenyl-C1-4 alkyl), and aryl alkenyl groups (such as C8-10 aryl alkenyls (such as cinnamyl), and phenyl-C2-4 alkenyl).

Another substituent group on the amino group is, for example, a heterocyclic group. Examples of the heterocyclic group substituted on the amino group include those similar to the heterocyclic groups described below.

Examples of the other substituent groups on the amino group include imidoyl groups (such as C1-6 alkylimidoyls (such as formylimidoyl and acetylimidoyl) and C1-6 alkoxyimidoyls, and C1-6 alkylthioimidoyls, and amidino).

The two substituent groups above may form a cyclic amino group together with a nitrogen atom. Examples of such cyclic amino groups include three-to eight-membered cyclic amino groups such as 1-piperazinyl, 1-pyrrolyl, and 1-imidazolyl. Such a cyclic amino group may be 1-azetidinyl, 1-pyrrolidinyl, pyperidino, thiomorpholino, morpholino, I-piperazinyl, or a cyclic amino group having a lower alkyl (such as C1-6 alkyl (such as methyl, ethyl, propyl, isopropyl, butyl, t-butyl, pentyl, or hexyl)), an aralkyl (such as benzyl or a C7-10 aralkyl(such as phenethyl)), or an aryl (such as C6-10 aryls (such as phenyl, 1-naphthyl, or 2-naphthyl)) substituted at the 4 position.

(2) Examples of the heterocyclic group include heterocyclic aromatic groups, saturated or unsaturated nonaromatic heterocyclic groups, and the like containing one or more of oxygen, nitrogen and sulfur atoms as the ring constituents. The heterocyclic groups include widely those having part of the chemical structure substituted not only on one site but also on multiple sites.

Examples of the heterocyclic aromatic groups include five- to six-membered monocyclic heterocyclic aromatic groups (such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, and triazinyl) and eight- to four-membered fused polycyclic heterocyclic aromatic groups (such as benzofuranyl, isobenzofuranyl, benzo[b]thienyl, indolyl, isoindolyl, 1H-indazolyl, benzindazolyl, benzoxazolyl, 1,2-benzisooxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphtyridinyl, purinyl, pteridinyl, carbazolyl, α-carbolinyl, β-carbolinyl, y-carbolinyl, acridinyl, phenoxazinyl, phenothiazinyl, phenazinyl, phenoxathiinyl, thianthrenyl, phenatrizinyl, phenatrolinyl, indolidinyl, pyrrolo[1,2.b]pyridazinyl, pyrazolo[1,5-alpyridyl, imidazo[1,2-b]pyridyl, imidazo[1,5-a]pyridyl, imidazo[1,2-b]pyridazinyl, imidazo[1,2-a]pyrimidinyl, 1,2,4-triazolo[4,3-a]pyridyl, and 1,2,4-triazolo[4,3-b]pyridazinyl).

Examples of the nonaromatic heterocyclic groups include three- to eight-membered saturated or unsaturated nonaromatic heterocyclic groups (such as oxiranyl, azetidinyl, oxetanyl, thietanyl, pyrrolidinyl, tetrahydrofuryl, thiolanyl, pyperidyl, tetrahydropyranyl, morpholinyl, thiomorpholinyl, and piperazinyl), monocyclic heterocyclic aromatic groups (such as 1,2,3,4-tetrahydroquinolyl and 1,2,3,4-tetrahydroisoquinolyl), nonaromatic heterocyclic groups having a fused polycyclic heterocyclic aromatic group, part or all double bonds of which are saturated, and the like.

Examples of the substituent groups on the heterocyclic group include lower alkyl groups (e.g., C1-6 alkyls (such as methyl, ethyl, and propyl)), lower alkenyl groups (e.g., C2-6 alkenyls (such as vinyl and allyl)), lower alkynyl groups (e.g., C2-6 alkynyls (such as thynyl and propargyl)), acyl groups (e.g., C1-6 alkanoyls (such as formyl, acetyl, propionyl, and pivaloyl), and benzoyl), amino groups (including those having a chemical structure partially substituted), hydroxyl groups (including those having a chemical structure partially substituted), halogen atoms (such as fluorine, chlorine, bromine, and iodine), imidoyl groups (including those having a chemical structure partially substituted), amidino groups (including those having a chemical structure partially substituted), and the like.

(3) The alicyclic group is, for example, a saturated or unsaturated alicyclic hydrocarbon group such as cycloalkyl, cycloalkenyl, or cycloalkadienyl.

Examples of the cycloalkyl groups include C3-9 cycloalkyl groups (such as cyclopropyl, cyclobutyl, cyclopentyl, cyclohexyl, cycloheptyl, cyclooctyl, and cyclononyl) and the like.

Examples of the cycloalkenyl groups include C3-6 cycloalkenyl groups (such as 2-cyclopenten-1-yl, 3-cyclopenten-1-yl, 2-cylcohexen-1-yl, 3-cylcohexen-1-yl, 1-cyclobuten-1-yl, and 1-cyclopenten-1-yl) and the like.

Examples of the cycloalkadienyl groups include C4-6 cycloalkadienyl groups (such as 2,4-cyclopentanedien-1-yl, 2,4-cyclohexanedien-1-yl, and 2,5-cyclohexanedien-1-yl) and the like.

Examples of the aromatic hydrocarbon groups include phenyl, naphthyl (1-naphthyl or 2-naphthyl), anthryl (1-anthryl or 2-anthryl), azulenyl, phenanthryl, phenanthrenyl, fluorenyl, indacenyl, biphenylenyl, heptalenyl, acenaphthylenyl and the like.

Hereinafter, chemical formulae of the compounds having -(CH₂)ₙ-R² as R¹, examples of the compounds according to the present invention, will be shown as Formulae 11 to 17. Among the groups, a m-ditrifluorophenyl group ("Formula 17") is particularly favorable. In each of the compounds, n is an integer of 1 to 15, more preferably of 1 to 5.

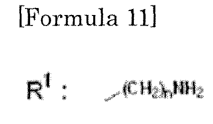

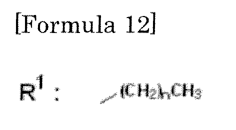

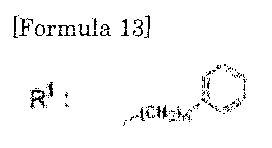

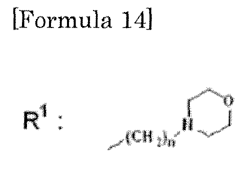

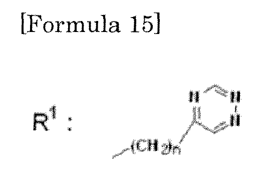

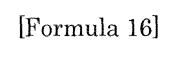

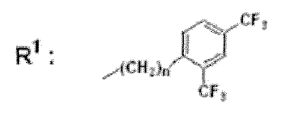

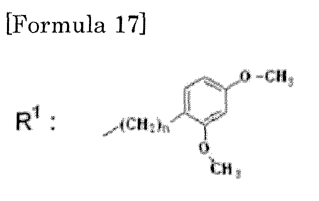

As described above, the compounds according to the present invention include the pharmaceutically allowable salts and solvated products of the compounds above. Examples of the salts favorably used include alkali-metal salts (such as sodium salt, potassium salt, and lithium salt), alkali-earth metal salts (such as calcium salt and magnesium salt), metal salts (such as aluminum salt, iron salt, zinc salt, copper salt, and nickel salt), inorganic salts (such as acetate salt and ammonium salt), organic amine salts (dibenzylamine salt, glucosamine salt, ethylenediamine salt, diethylamine salt, triethylamine salt, dicyclohexylamine salt, diethanol amine salt, and tetramethylammonium salt), and amino acid salts (such as glycine salt, lysine salt, arginine salt, ornithine salt, and asparagine salt).

### <Synthetic method for the compounds according to the present invention>

The synthetic method for the compounds according to the present invention is not particularly limited, and it is obtained, for example, by isolating and purifying VST from the culture supernatant of a VST-producing microbe (see Patent Document 1) and substituting a particular ketone group in the chemical structure of VST (introducing an organic group into the ketone group unit).

The organic group may be introduced by any known method and thus, the method is not particularly limited. For example, it is possible to prepare the compounds according to the present invention by introducing an organic group into a particular ketone group of VST, by preparing an oxime amine derivative having an organic group and allowing coupling of the oxime amine derivative with VST.

The coupling reaction between the oxime amine derivative and VST is performed, for example, by adding and dissolving the oxime amine derivative and VST in an organic solvent and leaving the solution in the presence of a small amount of added acetic acid at around 50°C for a particular time. Alternatively, the compounds obtained may be further, for example, dissolved in a solvent and reduced with a reducing agent, to give a final product, depending on the compounds to be prepared. The product obtained may be purified, for example, by HPLC.

The oxime amine derivative can be prepared by a known method. For example, an oxime amine derivative having an alkyl chain such as hexadecyl or octadecyl can be prepared easily by using the corresponding alcohol (hexadecanol or octadecanol) as the starting material.

### <Pharmaceutical composition according to the present invention>

The pharmaceutical composition according to the present invention is not particularly limited, if it contains at least the compounds according to the present invention.

The preparation of the pharmaceutical composition according to the present invention is not particularly limited. For example, it may be a solid preparation (such as tablet, capsule, granule, or powder) or a liquid preparation (such as sirup or injection).

A pharmaceutically allowable carrier may be used in blending the compounds according to the present invention in a pharmaceutical composition. Various organic or inorganic carrier substances commonly used as raw materials for such preparations are used favorably.

For example, in the case of a solid preparation, compounds such as diluent, lubricant, binder, and disintegrant are added additionally to the compounds according to the present invention and the carrier. In the case of a liquid preparation, compounds such as solvent, solubilizing agent, suspending agent, isotonic agent, buffer agent, and soothing agent are added as needed to the compounds according to the present invention and the carrier. In addition, additives for such preparations such as antiseptic, antioxidant, colorant, and sweetener may also be used.

Favorable examples of the diluents for use include lactose, sucrose, D-mannitol, starch, crystalline cellulose, light anhydrous silica and the like.

Favorable examples of the lubricants for use include magnesium stearate, calcium stearate, talc, colloidal silica, and the like.

Favorable examples of the binders for use include crystalline cellulose, sucrose, D-mannitol, dextrin, hydroxypropylcellulose, hydroxypropylmethylcellulose, polyvinylpyrrolidone, and the like.

Favorable examples of the disintegrants for use include starch, carboxymethylcellulose, carboxymethylcellulose calcium, croscarmellose sodium, carboxymethylstarch sodium, and the like.

Favorable examples of the solvents for use include injection water, alcohol, propylene glycol, macrogol, benne oil, corn oil, and the like.

Favorable examples of the solubilizing agents for use include polyethylene glycol, propylene glycol, D-mannitol, benzyl benzoate, ethanol, trisaminomethane, cholesterol, triethanolamine, sodium carbonate, sodium citrate, and the like.

Favorable examples of the suspending agents for use include surfactants (such as stearyltriethanolamine, sodium laurylsulfate, laurylaminopropionic acid, lecithin, benzalkonium chloride, benzethonium chloride, and glycerin monostearate), hydrophilic polymers (such as polyvinylalcohol, polyvinylpyrrolidone, sodium carboxymethylcellulose, methylcellulose, hydroxymethylcellulose, hydroxyethylcellulose, and hydroxypropylcellulose), and the like.

Favorable examples of the isotonic agents for use include sodium chloride, glycerol, D-mannitol, and the like.

Favorable examples of the buffer agents for use include buffer solutions of a phosphate salt, an acetate salt, a carbonate salt, a citrate salt, or the like.

Favorable examples of the soothing agents for use include benzyl alcohol and others.

Favorable examples of the antiseptics for use include paraoxybenzoate esters, chlorobutanol, benzyl alcohol, phenethyl alcohol, dehydroacetic acid, sorbic acid, and the like.

Favorable examples of the antioxidants for use include sulfite salts, ascorbic acid, and the like.

In addition, the pharmaceutical composition may contain another active ingredient, for example another anti-cancer agent (such as chemical therapy agent, immunotherapeutic agent, cell growth factor, or inhibitor to its receptor action).

The pharmaceutical composition according to the present invention contains the compounds according to the present invention in an amount normally of 0.1 to 95% (W/W) in the entire preparation, although the content may vary, depending on the preparation, the administration method, the carrier and others thereof, and the composition is prepared by a common production method.

### <Indications of the pharmaceutical composition according to the present invention>

Potentially, the pharmaceutical composition according to the present invention may be applied, for example, as an anti-cancer agent.

For example, the pharmaceutical composition according to the present invention can possibly be applicable to various cancers (such as breast cancer, prostate cancer, pancreatic cancer, stomach cancer, lung cancer, colon cancer, rectal cancer, esophageal cancer, duodenal cancer, tongue cancer, pharyngeal cancer, brain tumor, neurilemmoma, non-small-cell lung cancer, lung small cell carcinoma, liver cancer, renal cancer, biliary cancer, uterine body cancer, cervical cancer, ovarian cancer, bladder cancer, skin cancer, hemangioma, malignant lymphoma, malignant melanoma, thyroid cancer, osteoncus, angiofibroma, retinal sarcoma, penis cancer, infantile solid cancer, Kaposi sarcoma, AIDS-derived Kaposi sarcoma, maxillary sinus tumor, fibrous histiocytoma, smooth muscle sarcoma, striated muscle sarcoma, and leukemia).

The pharmaceutical composition according to the present invention can be administered to mammals (e.g., human, horse, bovine, dog, cat, rat, mouse, rabbit, porcine, simian, etc.).

The pharmaceutical composition can be administered, for example, orally as tablet, capsule (also as soft capsule or microcapsule), powder, or granule, or parenterally as injection, suppository, or pellet. The parenteral administration includes administration into vein, muscle, hypodermis, organ, nasal cavity, intradermis, eye, brain, rectum, vagina, abdominal cavity, tumor, an organ close to tumor and others, and also administration directly to the seat of disease.

The dosage of the compounds according to the present invention may vary according to the administration route, symptom and others, but, in the case of oral administration to patients (body weight: 40 to 80 kg), it is, for example, 0.5 to 100 mg/kg-body weight per day, preferably 1 to 50 mg/kg-body weight per day. For example, a preparation in the amount may be administered all at once or three times as divided every day.

The pharmaceutical composition may be used alone or in combination with another anti-cancer agent, for example, according to purpose, application, and symptom.

### Example 1

In Example 1, the compounds having the chemical structure represented by "Formula 11" as the substituent group R¹ (n=3) was prepared.

First, an oxime amine derivative was prepared (see the Reaction Formula of "Formula 18").

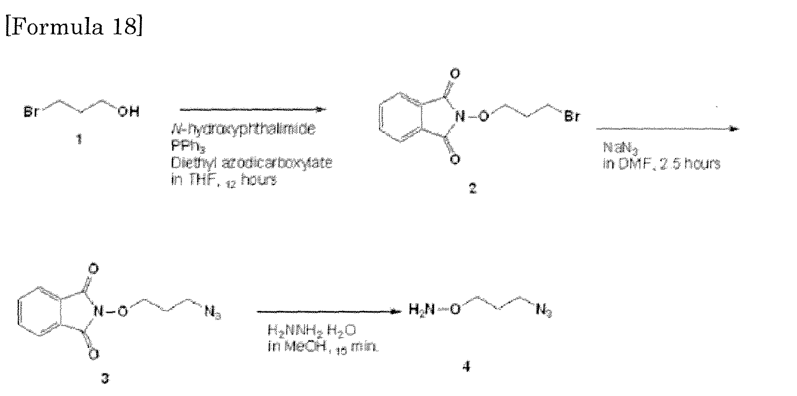

3-Bromo-1-propanol (3.0 g, the compounds No. 1), N-hydroxyphthalimide (3.6 g), and PPh₃ (5.7 g) were used as the starting materials. These starting materials were dissolved in dry THF (100 ml); diethylazodicarboxylate (3.6 ml) was added thereto; and the mixture was stirred overnight. After reaction, the mixture was concentrated and purified by silica gel chromatography (hexane:ethyl acetate: 10:1, or benzene:ether: 10:1), to give the compounds No. 2.

Then, the compounds No. 2 (3.0 g) was dissolved in dry DMF (50 ml); NaN₃ (1.3 g) was added thereto; and the mixture was stirred for 2.5 hours. Then, the reaction solution was partitioned between distilled water and ethyl acetate, and the organic layer was dried over MgSO₄ and concentrated to dryness. The concentrate was then purified by silica gel chromatography (hexane:ethyl acetate: 10:1, or benzene:ether: 10:1), to give the compounds No. 3.

The product was then dissolve in methanol (30 ml); the mixture was treated with H₂ NNH₂ H₂O (850 mg) for 15 minutes, and then concentrated to dryness and purified by silica gel chromatography (hexane:ethyl acetate: 5:1, or 3:1), to give an oxime amine derivative (3-aminooxypropyl-1-azide, compounds No. 4).

Subsequently, an organic group was introduced into a particular ketone group site in the VST chemical structure, in coupling reaction between VST and the oxime amine derivative (see the Reaction Formula of "Formula 19").

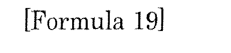

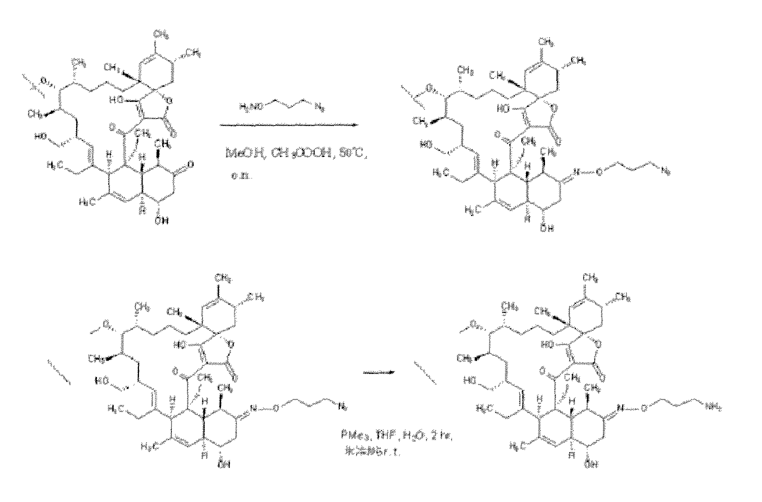

The 3-aminooxypropyl-1-azide obtained (22 mg) and VST (200 mg) were dissolved in methanol (10 ml); a drop of acetic acid was added thereto; and the mixture was allowed to react at 50°C overnight. After concentration to dryness, the solid was dissolved in THF (5 ml); a drop of distilled water was added; and the mixture was ice-cooled. After addition of trimethylphosphine (200 µl), the mixture was stirred for 2 hours while gradually warmed to room temperature, and after reaction, the product was purified by reversed-phase HPLC (75% methanol), to give the desired compounds.

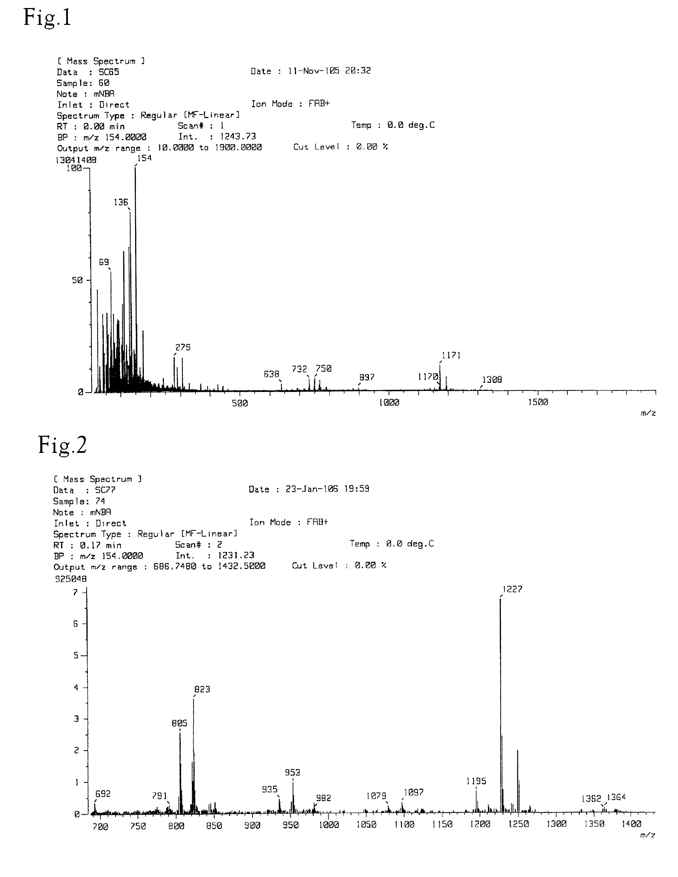

The compounds obtained were subjected to mass spectrometric analysis. The results are shown in Figure 1. The molecular weight was 1171.50.

### Example 2

In Example 2, an example of the synthetic method for the compounds having the chemical structure represented by "Formula 12" as the substituent group R¹ (n=3) will be described.

The corresponding alcohol is used as the starting material. An oxime amine derivative is prepared according to the Reaction Formula shown in "Formula 20". VST and the oxime amine derivative were then allowed to react in coupling reaction, in a similar manner to Example 1, to give the desired compounds.

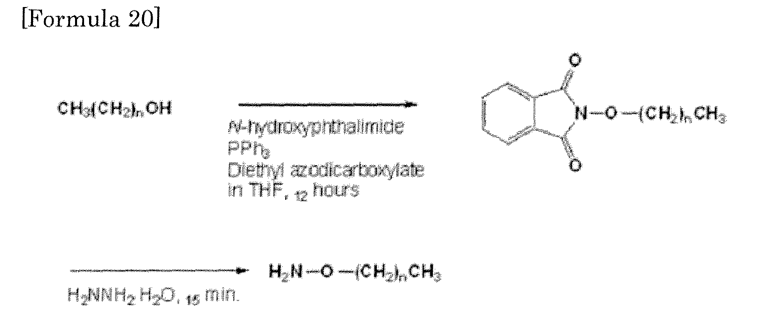

### Example 3

In Example 3, the compounds having the chemical structure represented by "Formula 14" as the substituent group R¹ (n=2) was prepared.

An oxime amine derivative was first prepared according to the Reaction Formula of "Formula 21". Then, VST and the oxime amine derivative were allowed to react in coupling reaction, in a similar manner to Example 1, to give the desired compounds.

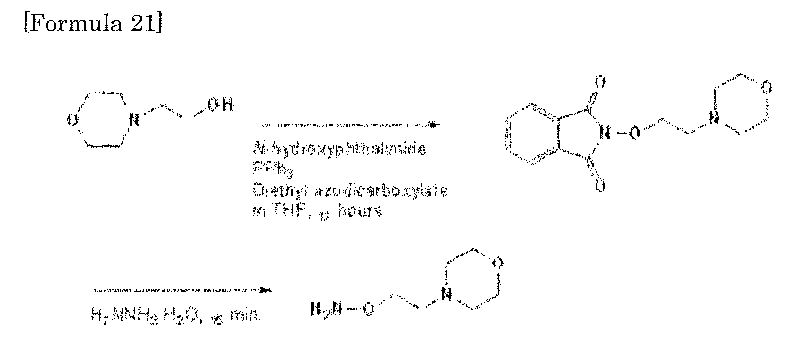

The compounds obtained were subjected to mass spectrometric analysis. The results are shown in Figure 2. The molecular weight was 1227.56.

### Example 4

In Example 4, the compounds having the chemical structure represented by "Formula 16" as the substituent group R¹ (n=3) was prepared.

The compounds were prepared in a similar manner to Example 1.

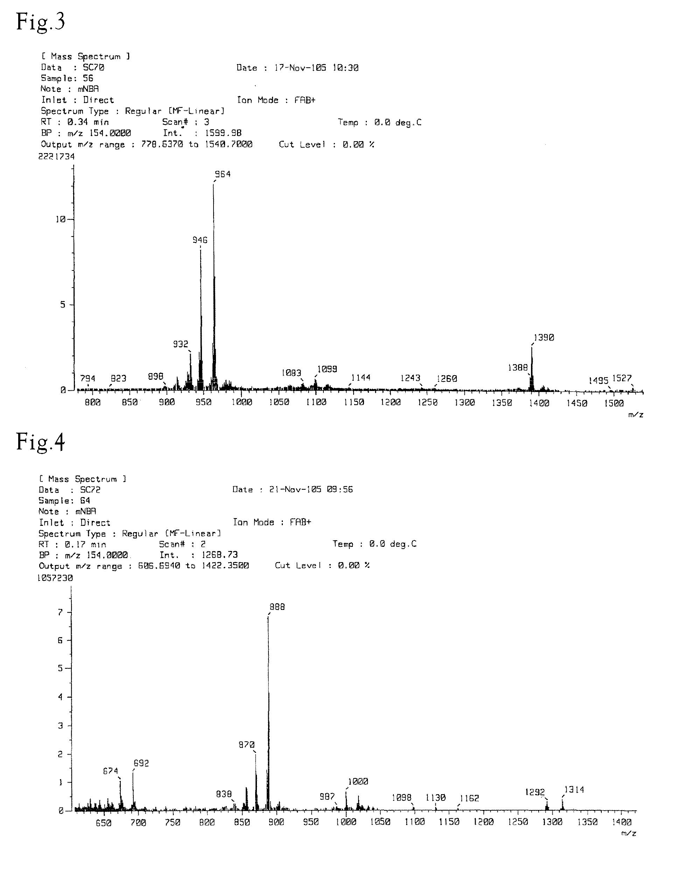

The compounds obtained were subjected to mass spectrometric analysis. The results are shown in Figure 3. The molecular weight was 1368.58.

### Example 5

In Example 5, the compounds having the chemical structure represented by "Formula 17" as the substituent group R¹ (n=3) was prepared.

The compounds were prepared in a similar manner to Example 1.

The compounds obtained were subjected to mass spectrometric analysis. The results are shown in Figure 4. The molecular weight was 1292.63.

### Example 6

In Example 6, the action of the VST derivative to inhibit expression of GRP 78 was studied by Western blot method. In the present experiment, the compounds prepared in Example 1 (the compounds having the chemical structure represented by "Formula 11" as the substituent group R¹ (n=3)) was used as the VST derivative.

Expression of GRP 78 is known to induce under glucose-deficient condition. Thus in the present experiment, the action of the VST derivative to induce or inhibit expression of GPR78 was studied both under glucose-deficient condition and also under normal condition.

The experiment was performed generally in the following manner: First, HT 1080 cell (cancer cell) was cultured in RPMI 1640 medium. Then in the case of the glucose-deficient condition, the medium was removed and substituted with glucose(-) RPMI 1640 medium; VST or the VST derivative was added to the medium to a particular concentration; and the mixture was cultured for 18 hours (in the case of the normal condition, after medium exchange, VST or the VST derivative was added to a particular concentration, and the RPMI 1640 medium contained glucose at a concentration of 2 g/L). Then, cultured cells were harvested, and GRP 78 expressed was detected by Western blotting with an anti-GRP 78 antibody.

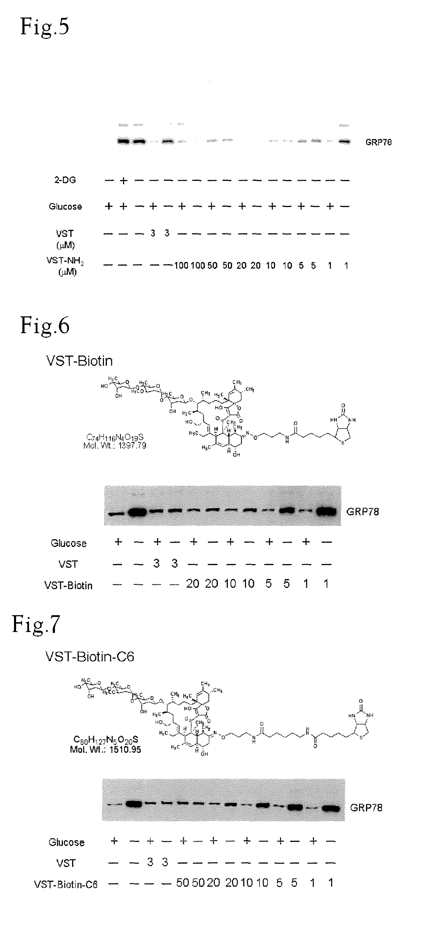

The results are shown in Figure 5. Figure 5 is a Western blot photograph showing the expression amount of GRP 78.

In the Figure, the first to fifth lanes from the left show the results obtained in control and Comparative Examples. The far left lane shows a band showing the GRP 78 expression amount when glucose is added; the second lane from the left shows a band showing the GRP 78 expression amount in the glucose-deficient state artificially generated by addition of 2-deoxyglucose; and the third lane from the left shows a band showing the GRP 78 expression amount in the absence of glucose (in the case of glucose-deficient state). As shown in Figure 5, expression of GRP 78 is induced in the case of the glucose-deficient state (the second and third lanes from the left), compared to the case in the presence of glucose (the far left lane). The fourth and fifth lanes from the left show band showing the GRP 78 expression amount when VST was added. As indicated by the fifth lane from the left, induction of GRP 78 expression is inhibited to some extent even in the glucose-deficient state, when VST was added.

The sixth and following lanes from the left show the inhibitory action to induction of GRP 78 expression observed when the VST derivative according to the present invention was added. As shown by the Western blot photograph of respective lanes, the expression amount of GRP 78 in the lanes in the absence of glucose was almost the same as that in the lanes in the presence of glucose. The results indicate that the VST derivative according to the present invention inhibited induction of GRP 78 expression, similarly to (or more efficiently than) VST, under the glucose-deficient condition. The results in the present experiment also suggest that the VST derivative having an organic group introduced on a particular ketone group site in the VST chemical structure has an action to induce GRP 78 expression, similarly to VST.

### Example 7

In Example 7, biotins with various spacers were introduced to a particular ketone group site in the VST chemical structure, and the action to inhibit GRP 78 expression by the VST derivatives was studied by the Western blot method.

First, the VST oxime amine derivative prepared in Example 1 and each compounds represented by the general formula of "Formula 22" were allowed to react in DMF in the presence of an acid or alkali (such as acetic acid or triethylamine), to give three kinds of VST biotin derivatives.

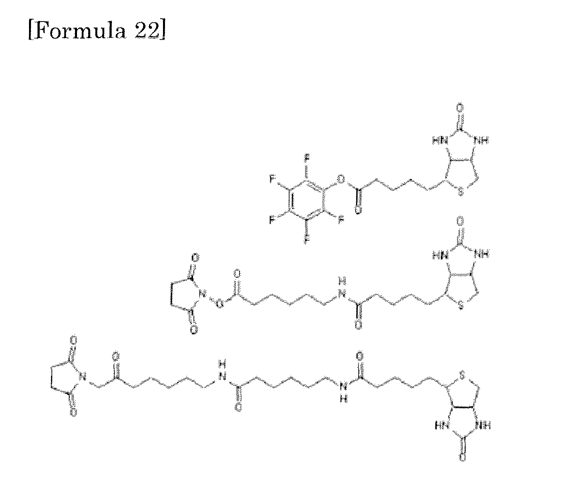

Then, the cell was cultured in a similar manner and condition to those in Example 6, and GRP 78 expressed was detected by Western blotting by using an anti-GRP 78 antibody.

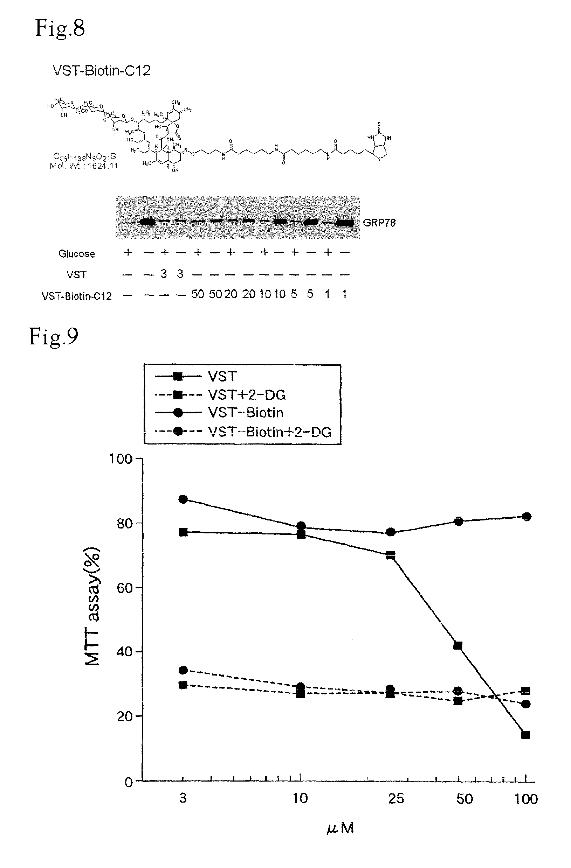

The results are shown in Figures 6 to 8. Figure 6 is a Western blot photograph obtained when a derivative having biotin introduced at a particular ketone group site in the VST chemical structure was used; Figure 7, when a similar derivative having a C6-biotin introduced was used; and Figure 8, when a similar derivative having C12-biotin introduced was used. The Chemical Formulae of the VST biotin derivatives used in the experiment above are also shown respectively in the Figures. In addition in Figures 6 to 8, the first to fourth lanes represent controls similar to those in Example 6.

The fifth and following lanes in Figures 6 to 8 show the inhibitory action to induction of GRP 78 expression when each of the VST derivatives was added. For example, the Western blot photographs of the sixth and eighth lanes from the left in Figure 6 show that the VST biotin derivatives inhibit induction of GRP 78 expression, similarly to VST under the glucose-deficient condition (also in Figures 7 and 8). In addition, the results above strongly suggest that the VST derivative having an organic group introduced at a particular ketone group site in the VST chemical structure has an action to induce GRP 78 expression, similarly to VST, independently of the length of the carbon chain of the organic group.

### Example 8

In Example 8, the cell survival rate when a VST derivative was added was studied by a MTT assay. The VST derivatives used were the three kinds of VST biotin derivatives prepared in Example 7.

The experiment was performed generally in the following manner: A cell suspension of HT 1080 cell (5×10⁴/ml) was placed in each well on a 96-well plate in an amount of 100 µl, and incubated at 37°C for 6 hours; solutions of VST or a VST derivative at varying concentrations were added thereto; and the mixtures were incubated for 24 hours. 20 mM of 2-DG (deoxyglucose) was also added when VST or the VST derivative was added, to bring the system into a glucose-deficient state. Then after incubation for 24 hours, 10 µl of 0.05 g/ml MTT solution was added to each well, and the mixture was incubated at 37°C for 4 hours. The medium was then removed; 100 µl of DMSO was added; and then, the absorbance at 530 nm was determined, to give a relative value of the surviving cell count.

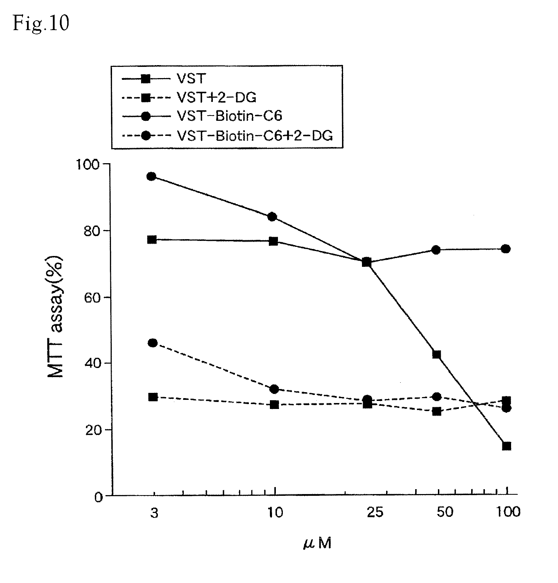

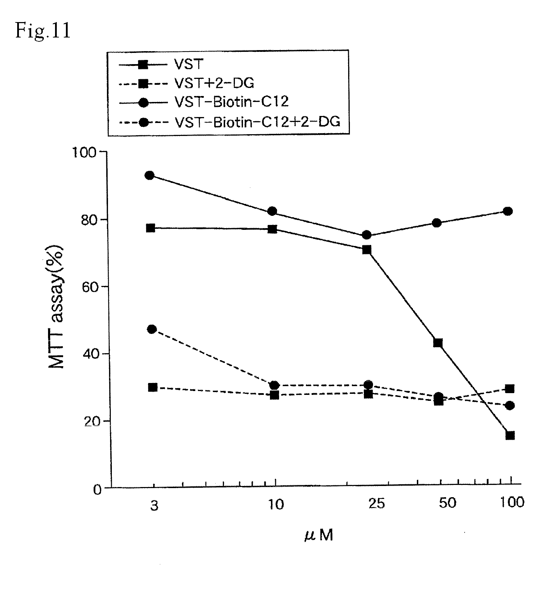

The results are shown in Figures 9 to 11. Figure 9 is a graph showing the cell survival rate obtained when a derivative having biotin introduced at a particular ketone group site in the VST chemical structure was used as the VST derivative; Figure 10, when a derivative having the C6-biotin introduced similarly to a particular ketone group site; and Figure 11, when a derivative having the C12-biotin introduced similarly to a particular ketone group site. The abscissa of the graph shows the concentration (logarithm) of each VST or the VST derivative added, while the ordinate, the cell survival rate (%) as determined in the MTT assay. In each graph, "VST" represents a sequential line graph obtained when VST was added;
"VST+2-DG", when VST and 2-DG were added, i.e., when VST was added in the glucose-deficient state; "VST-Biotin", "VST-Biotin-C6", and
"VST-Biotin-C12", respectively sequential line graphs when respective VST derivatives were added; "VST derivative + 2-DG", when each VST derivative and 2-DG were added, i.e., when the VST derivative and 2-DG were added in the glucose-deficient state.

In the present experiment, the cell survival rate when 20 mM of 2-DG was added to HT 1080 cell to bring the system into the glucose-deficient state was approximately 50% (not shown in the figure). In contrast, the cell survival rate when VST or a VST derivative and 2-DG were added was lower at around 30%, as shown in any one of Figures 9 to 11. The results suggest that VST or the VST derivative inhibits growth of the cancer cell.

On the other hand, as shown in Figures 9 to 11, the cell survival rate declined significantly when VST was added, especially when the VST addition amount exceeded 25µM, in the case where 2-DG was not added, while the cell survival rate did not declined particularly, when the VST derivative was added, independently of the kind of the derivative, even if the addition amount was increased. The results strongly suggest that it is possible to reduce the cell toxicity significantly by introducing a substituent on a particular ketone group of VST, and that the VST derivative according to the present invention has smaller cell toxicity and adverse reaction and is thus superior in safety.

### Example 9

In Example 9, inhibition of GRP 78 expression by the VST derivative was studied by a luciferase assay. The VST derivatives used were four kinds of VST derivatives prepared respectively in Examples 1, 3, 4, and 5.

The "luciferase assay" will be described below briefly. The luciferase is an enzyme protein catalyzing bioluminescence. For example, when a recombination gene including a gene coding the promoter region of a particular gene and that of luciferase is produced and integrated into a culture cell, induction of expression of the gene leads to additional expression of luciferase. Thus, measurement of the bioluminescence by luciferase and determination of the luciferase expression level allows estimation of the expression induction level of the gene.

The experimental procedure will be described briefly below.

First, the GRP 78 promoter region was integrated with a luciferase emission vector pGL3 basic (manufactured by Promega), to give a recombination vector. The recombination vector has the GRP 78 promoter region and a region coding the luciferase gene.

Then, the recombination vector prepared was transfected into culture cell HT 1080. The transfected culture cell was then incubated in the presence of a VST derivative and 2-DG (20 mM), allowing expression of luciferase. 36 hours after transfection, the subcultured cells were collected and suspended.

Then, a cell suspension was prepared by using Dual Luciferase Assay System (manufactured by Promega), and the light intensity was determined, to give the expression induction level of GRP 78.

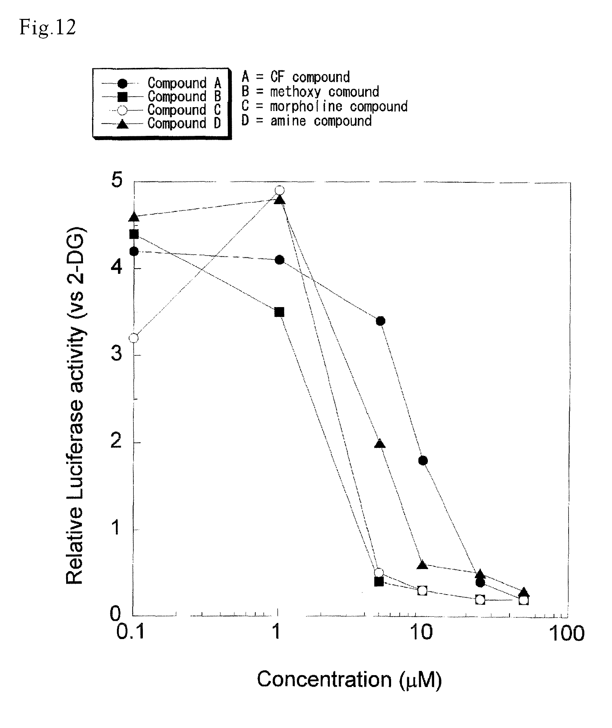

Results are summarized in Figure 12. Figure 12 is a graph showing the fluorescence intensity when a VST derivative was added.

The abscissa of the graph (Concentration (µM)) shows the concentration of the VST derivative, while the ordinate (Relative Luciferase activity), the light intensity. The light intensity is a relative value compared to "1" of the light intensity obtained when a plasmid containing only the luciferase gene was transfected to the culture cell. In the graph, "VS 2-DG" indicates that 2-DG was added.

In the graph, "Compound A (CF compounds)" indicates the results obtained when the VST derivative prepared in Example 4 was added; "Compound B (methoxy compounds)", when the VST derivative prepared in Example 5 was added; "Compound C (morpholine compounds)" when the VST derivative prepared in Example 3 was added; and "Compound D (amine compounds)" when the VST derivative prepared in Example 1 was added.

The results in Figure 12 indicate that the VST derivative inhibits expression of GRP 78.

### Example 10

In Example 10, the anti-cancer action at the cell level when a VST derivative was added to a culture cancer cell was studied.

HT 1080 cell was cultured in a 6-cm dish until the cells became 70% confluent, and VST or a VST derivative was added to a concentration of 10, 3, or 1 µM in the absence of glucose (in the glucose-deficient state), and the action thereof was studied. The VST derivative prepared in Example 1 (the compounds having the chemical structure (n=3) represented by "Formula 11" as the substituent group R¹) and the three kinds of VST biotin derivatives prepared in Example 7 were used as the VST derivatives.

As a result, the cell count decreased in any case where a VST derivative was added in the glucose-deficient state, indicating that the VST derivative had an action to inhibit cancer cell also at the cell level, similarly to VST.

### Example 11

In Example 11, the toxicity of the VST derivative was studied.

In the present Example, the VST derivative prepared in Example 1 (the compounds having the chemical structure represented by "Formula 11" as the substituent group R¹ (n=3)) was used as the VST derivative. The experiment procedure is essentially the same as that used in Example 10.
In the present Example, VST or the VST derivative was added at a concentration of 100 µM.

As a result, in normal culture environment (glucose in the presence of), addition of VST at a concentration of 100 µM resulted in significant decrease in cell count, while addition of the VST derivative in the same amount resulted in no particular decrease in cell count, indicating that the VST derivative is less toxic than VST.

On the other hand, when the cultured cell is in the glucose-deficient state, addition of the VST derivative resulted in cell death, similarly to addition of VST, indicating that the VST derivative had an action to inhibit growth of cancer cell at the cell level, similarly to VST.

### Example 12

In Example 12, the toxicity of the VST derivatives when the ketone group in the VST chemical structure is substituted with a biotin having a varying spacer was studied. In the present Example, the culture cell was brought into the glucose-deficient state by addition of 2-deoxyglucose.

The experiment procedure is essentially the same as that used in Example 10. The three kinds of VST biotin derivatives prepared in Example 7 were used as the VST derivatives. In the present Example, VST or the VST derivative was added to a concentration of 100µM, and 2-DG, to a concentration of 20 mM.

As a result, under normal culture environment, addition of VST at 100 µM resulted in significant drop in cell count, similarly to Example 11, while addition of the VST derivative in the same amount resulted in no significant drop in cell count, indicating that the VST derivative was less toxic than VST and that it was thus possible to reduce the toxicity, independently of the length of the carbon chain of the substituent group.

On the other hand, when the cultured cell was in the glucose-deficient state, addition of the VST derivative resulted in cell death, similarly to addition of VST, indicating that the VST derivative had an action to inhibit growth of cancer cell similarly to VST and that it had an action to inhibit growth of cancer cell also at the cell level, independently of the length of the carbon chain of the substituent group.

### Example 13

In Example 13, the toxicity test of the VST derivative was performed by using mice.

The mice used were 7- to 8-week old male ICR mice (available from Charles River). VST or a VST derivative was administered to each mouse by tail vein injection, and the toxicity thereof was studied. In the present experiment, the VST biotin derivative prepared in Example 7 was used as the VST derivative.

Results are summarized in Table 1.

**[Table 1]**

| Acute toxicity experiment | Dosage | Number of mice administered | Number of dead animals (within 24 hours) |
|---|---|---|---|
| VST | 6mg/kg | 5 | 0 |
| | 9 mg/kg | 5 | 0 |
| | 12 mg/kg | 6 | 1 |
| | 15 mg/kg | 6 | 5 |
| | 18 mg/kg | 4 | 4 |
| | 21 mg/kg | 4 | 4 |
| | 30 mglkg | 4 | 4 |
| VST derivative | 30 mg/kg | 6 | 0 |
| | 45 mg/kg | 3 | 0 |

As shown in Table 1, VST lead to death of all mice at a dosage of more than 18 mg/kg, while the VST derivative lead to no death of the mice even at a dosage of 45 mg/kg. The experimental results indicate that the VST derivative is significantly less toxic than VST at the individual level.

### Industrial Applicability

The VST derivative according to the present invention is applicable as an anti-cancer agent weaker in its adverse reaction and higher in safety.

### BRIEF DESCRIPTION OF THE DRAWINGS

Figure 1 is a mass spectrum of the compounds prepared in Example 1 (the compounds having the chemical structure represented by "Formula 11" as the substituent group R¹ (n=3)).
Figure 2 is a mass spectrum of the compounds prepared in Example 3 (the compounds having the chemical structure represented by "Formula 14" as the substituent group R¹ (n=2)).
Figure 3 is a mass spectrum of the compounds prepared in Example 4 (the compounds having the chemical structure represented by "Formula 16" as the substituent group R¹ (n=3)).
Figure 4 is a mass spectrum of the compounds prepared in Example 4 (the compounds having the chemical structure represented by "Formula 17" as the substituent group R¹ (n=3)).
Figure 5 is a Western blot photograph showing the induction of GRP 78 expression when the compounds prepared in Example 1 (the compounds having the chemical structure represented by "Formula 11" as the substituent group R¹ (n=3)) was used as the VST derivative in Example 6.
Figure 6 is a Western blot photograph showing the induction of GRP 78 expression when a derivative having biotin substituted on the VST ketone group was used as the VST derivative in Example 7.
Figure 7 is a Western blot photograph showing the induction of GRP 78 expression when a derivative having a C6-biotin substituted on the VST ketone group was used as the VST derivative in Example 7.
Figure 8 is a Western blot photograph showing the induction of GRP 78 expression when a derivative having a C12-biotin substituted on the VST ketone group was used as the VST derivative in Example 7.
Figure 9 is a graph showing the cell survival rate (results obtained in MTT assay) when a derivative having biotin substituted on the VST ketone group was used as the VST derivative in Example 8.
Figure 10 is a graph showing the cell survival rate when a derivative having biotin substituted on the VST ketone group was used as the VST derivative in Example 8 (results obtained in MTT assay).
Figure 11 is a graph showing the cell survival rate when a derivative having biotin substituted on the VST ketone group was used as the VST derivative in Example 8 (results obtained in MTT assay).
Figure 12 is a graph showing the fluorescence intensity when a VST derivative was added in Example 9 (results obtained in luciferase assay).

## Claims

1. The compounds represented by the general formula of "Formula 1" or the salt thereof 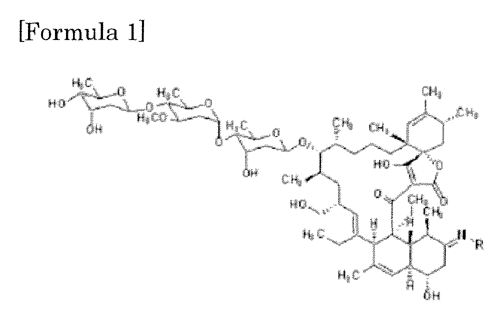 [wherein, R represents an organic group].

2. The compounds according to Claim 1, wherein R represents -O-R¹ [wherein, R¹ represents an organic group].

3. The compounds according to Claim 2, wherein R¹ represents a hydrocarbon group (that may be substituted).

4. The compounds according to Claim 2, wherein R¹ is a hydrocarbon group having a chemical structure, part of which is substituted with one or more of amino groups (that may be substituted), heterocyclic groups (that may be substituted), and alicyclic hydrocarbon groups (that may be substituted).

5. The compounds according to Claim 2, wherein R¹ represents -(CH₂)ₙ-R² [wherein, n is an integer of 1 or more, and R² represents a hydrogen atom, an amino group (that may be substituted), a heterocyclic group (that may be substituted), or an alicyclic hydrocarbon group (that may be substituted)].

6. The compounds according to Claim 5, wherein R² represents an acylamino group (that may be substituted), a phenyl group (that may be substituted), a morpholinyl group (that may be substituted), or a triazinyl group (that may be substituted).

7. The VST derivative according to Claim 2 or the salt thereof, wherein R¹ is one of the chemical structures represented by "Formulae 2 to 6":

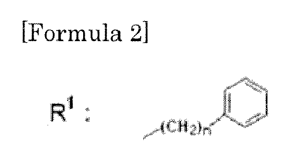

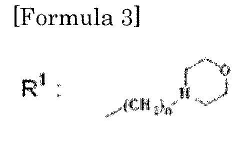

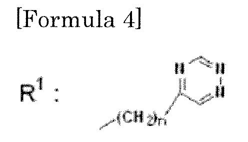

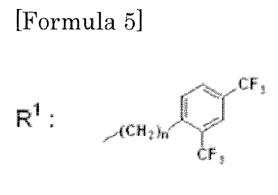

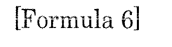

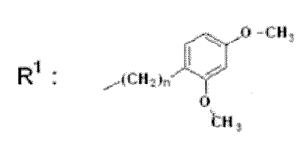

8. A pharmaceutical composition, comprising the compounds according to Claim 1, the salt thereof, or the prodrug thereof.

9. An anti-cancer agent, comprising the compounds according to Claim 1, the salt thereof, or the prodrug thereof.

10. An inhibitor to induction of GRP 78 expression that inhibits the induction of GRP 78 expression by microsomal stress, at least comprising the compounds according to Claim 1, the salt thereof, or the prodrug thereof.

11. A method of producing the compounds according to Claim 1, comprising a step of substituting a particular ketone group in the compounds represented by the general formula of "Formula 7":

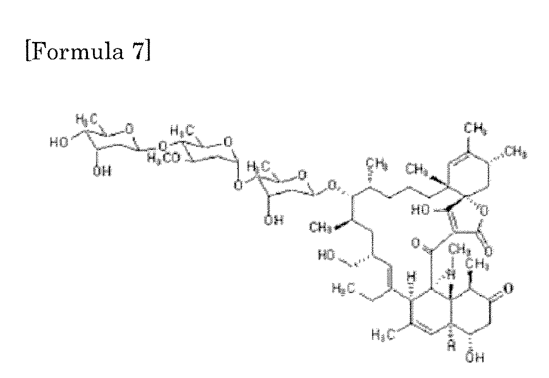

12. Use of the compounds according to Claim 1 or the salt or prodrug thereof for production of an anti-cancer agent.

13. A method of treating cancer, comprising administering an effective amount of the compounds according to Claim 1, the salt thereof, or the prodrug thereof.
